(19) Europäisches Patentamt / European Patent Office / Office européen des brevets

(11) **EP 4 042 970 A1**

(12) **EUROPEAN PATENT APPLICATION**

(43) Date of publication:
**17.08.2022 Bulletin 2022/33**

(21) Application number: **22167118.3**

(22) Date of filing: **12.06.2018**

(51) International Patent Classification (IPC):
**A61C 19/04** (2006.01)    **A61C 3/02** (2006.01)
**A61B 5/053** (2021.01)    **A61B 5/00** (2006.01)

(52) Cooperative Patent Classification (CPC):
**A61C 19/04; A61B 5/4547; A61C 3/02;**
A61B 2562/0271

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.06.2017 GB 201709640**

(62) Document number(s) of the earlier application(s) in accordance with Art. 76 EPC:
**18734879.2 / 3 638 149**

(71) Applicant: **Oxford University Innovation Limited Oxford, Oxfordshire OX2 0JB (GB)**

(72) Inventors:
• **CHANA, Kamaljit Singh**
  **Oxford, OX2 0ES (GB)**
• **SIHRA, Saranjit Singh**
  **Hornchurch, RM11 3JF (GB)**

(74) Representative: **J A Kemp LLP**
  **80 Turnmill Street**
  **London EC1M 5QU (GB)**

Remarks:
This application was filed on 07.04.2022 as a divisional application to the application mentioned under INID code 62.

(54) **DRILL**

(57)    A drill is disclosed that comprises a rotatable cutting surface and a sensor element positioned behind the rotatable cutting surface. The sensor element is configured to allow a heating pulse to be applied to a region in front of the cutting surface during rotation of the cutting surface in use and to allow measurement of a response of the sensor element during the heating pulse to determine chemical or structural information about the region in front of the cutting surface, the response being dependent on heat transfer characteristics of the region in front of the cutting surface.

## Fig.1

Processed by Luminess, 75001 PARIS (FR)

EP 4 042 970 A1

## Description

Bacteria collect and eat away at the material inside a tooth through a process of demineralization causing lesions, known as caries. Left untreated the lesions can lead to infections and cavities as well as to the loss of the tooth. Dentists need to detect the lesions. This includes detection of the extent of decay, detecting how far into the layers the decay has progressed, and detecting the distance between the decay and the nerve. Dentists also need to detect cracked teeth and be able to detect decay in between teeth and between or beneath obstacles such as silver fillings or crowns.

A dental probe comprising a handle and a rigid elongate member that can be manually pressed against or scraped over teeth to assess their state is widely used. Such probes are cheap but provide only limited and subjective information to the dentist.

X-rays can be used but expensive equipment is needed, the results are still subjective, and images can be blocked by structures that are relatively opaque to the X-rays, such as silver fillings or crowns. Furthermore, exposure of patients to X-rays has to be limited.

Laser light has been used to stimulate fluorescence where caries is present, but expensive equipment is still needed and existing methods can only be applied readily to easily accessible outer exposed surfaces of teeth, rather than in between the teeth.

Reliable detection and evaluation of gum disease is another important challenge for dentists. Gum disease can be monitored by measuring the depth of periodontal pockets, but this is difficult to assess objectively.

It is an object of the invention to provide alternative ways of obtaining information about the dental state of a patient that at least partially address one or more of the issues discussed above, or other issues.

According to an aspect of the invention, there is provided a method of sensing a dental region, comprising: using a sensor element to apply a heating pulse to the dental region; and determining chemical or structural information about the dental region by measuring a response of the sensor element during the heating pulse, the response being dependent on heat transfer characteristics of the dental region.

The method uses measurements of heat transfer characteristics to obtain information about the dental region. The method is non-invasive. The method can be implemented using relatively inexpensive, safe, and compact equipment. The method can detect dental problems before they become visible to X-rays and/or in situations where X-rays are blocked by opaque matter. The sensor element can be incorporated into the distal end of an otherwise conventional explorer probe or dental drill.

In an embodiment, the dental region comprises any surface or structure relevant to oral health, including one or more regions of one or more of the following: teeth, gums, oral mucosa, upper jaw, lower jaw, tongue, salivary glands, uvula, and frenulum. In an embodiment, the dental region comprises a portion of a tooth. Alternatively or additionally, the dental region comprises a periodontal pocket. Alternatively or additionally, the dental region comprises a region of jaw bone, for example for evaluating a state of a dental implant.

In an embodiment, the sensor element comprises a resistive element. In an embodiment the resistive element is a thin film resistive element, optionally comprising platinum or gold. Thin film resistive elements are naturally compact. When provided flat against a substrate the thin film element is robust mechanically, and can easily be protected by a thermally conductive protective layer, such as a layer of diamond-like carbon.

In an embodiment the resistive element is mounted on a substrate in such a way that at least 10% of the surface area of the resistive element is in contact with the substrate (e.g. as a thin film element mounted on a substrate). An advantage of this arrangement is that significant heating power can be applied to the resistive element without the resistive element reaching excessively high temperatures. The substrate acts to conduct heat effectively away from the resistive element.

In an embodiment, heat from the heating pulse propagates through plural layers of different chemical or structural composition and the measured response of the sensor element is analysed to identify one or more target time periods, each target time period being defined as a time period in which the response of the sensor element is determined predominantly by a different combination of one or more of the plural layers. Information about particular target layers in a multilayer structure can therefore be obtained. Perfect contact between the sensor element and the dental region to be sensed is not necessary because a contribution to the response of the sensor element from material between the sensor element and the dental region (in the case of imperfect contact) can be recognized and taken account of. In an embodiment, a coupling fluid or gel is provided between the sensor element and the dental region during application of the heating pulse to the dental region. The coupling fluid or gel helps reproducibly to provide a high quality thermal contact between the sensor element and the dental region being sensed.

The chemical or structural information may be presented to a user in the form of a graphical display. The graphical display may comprise an image of a tooth, for example. The image of the tooth may be updated in response to measurements using the sensor element, for example to show different portions of a cross-section of the tooth in different colours to indicated boundaries between portions of the tooth having different respective chemical or structural compositions. The image of the tooth may be updated in response to measurements using the sensor element pressed against a plurality of different respective portions of the tooth, optionally in a progressive manner to progressively build up a visual map of the interior of the tooth.

According to an aspect of the invention, there is provided a dental probe, comprising: a handle and a sensor element mounted distally relative to the handle, wherein: the probe is configured to allow a user holding the handle to bring the sensor element into thermal contact with a dental region to be sensed; and the sensor element is configured to allow a heating pulse to be applied to the dental region via the sensor element and to allow measurement of a response of the sensor element during the heating pulse to determine chemical or structural information about the dental region, the response being dependent on heat transfer characteristics of the dental region.

According to an aspect of the invention, there is provided a device for sensing multiple dental regions, comprising: a support structure configured to fit against a plurality of teeth of a patient, wherein: the support structure comprises a plurality of sensor elements; each sensor element is configured to be positioned against a respective different dental region when the support structure is positioned in use against the plurality of teeth; and each sensor element is configured to allow a heating pulse to be applied to the dental region against which the sensor element is positioned and to allow measurement of a response of the sensor element during the heating pulse to determine chemical or structure information about the dental region, the response being dependent on heat transfer characteristics of the dental region.

Thus, a device is provided which allows multiple measurements to be performed quickly, easily and reproducibly. Measurements made at different times can be compared to each other in a meaningful way and deviations detected with high sensitivity and reliability. Dental problems can be detected at an early stage.

According to an aspect of the invention, there is provided a drill comprising: a rotatable cutting surface and a sensor element positioned behind the rotatable cutting surface, wherein: the sensor element is configured to allow a heating pulse to be applied to a region in front of the cutting surface during rotation of the cutting surface in use and to allow measurement of a response of the sensor element during the heating pulse to determine chemical or structural information about the region in front of the cutting surface, the response being dependent on heat transfer characteristics of the region in front of the cutting surface.

Thus, a drill is provided that is capable of detecting the chemical and/or structural composition of material ahead of the drill tip. The risk of incomplete or excessive drilling can be reduced. The depth of decay can be monitoring during drilling. A separation between the drill and a nerve can be monitored during drilling.

Embodiments of the invention will now be described, by way of example only, with reference to the accompanying drawings in which corresponding reference symbols indicate corresponding parts, and in which:

Figure 1 is a schematic side view of a dental probe;

Figure 2 is a magnified perspective view of a distal portion of the dental probe of Figure 1;
Figure 3 is a graph depicting measured responses from a sensor element applied to different materials;
Figure 4 is a schematic sectional view depicting use of a dental probe to measure a depth of a periodontal pocket;
Figure 5 is a schematic top view depicting use of a device for sensing multiple dental regions;
Figure 6 is a schematic side view of a drill comprising a sensor element; and
Figure 7 depicts example circuitry for measuring a response of a sensor element to heating pulses.

Embodiments of the present disclosure provide methods of obtaining information about a dental region based on thermal measurements. The methods use a sensor element to apply a heating pulse to the dental region. A response of the sensor element during the heating pulse is analysed to determine heat transfer characteristics of the dental region. The heat transfer characteristics affect how efficiently heat will be conducted away from the sensor element. Heat from the heating pulse penetrates underneath the surface of the dental region being sensed (e.g. several millimetres into tooth material), allowing sub-surface structure to be sensed, such as caries or other abnormalities, as well as nerve position and the morphology or state of implanted material such as fillings. Sensing can be achieved effectively even for relatively low energy pulses. In the case of sensing teeth, for example, the method can be performed without increasing the temperature of the tooth by more than about two degrees Celsius. The small increases in tooth temperature that do occur last for only a short period, typically less than one second. The method does not cause any discomfort to the patient.

Heat transfer characteristics of materials (e.g. thermal properties such as thermal conductivity, $\kappa$; specific heat capacity, $c$, and quantities that depend on one or both of these properties) can depend sensitively on the composition (e.g. chemical or structural) of the materials. The thermal product, $\sqrt{\rho c \kappa}$, where $\rho$ is equal to the density, is often a heat transfer characteristic that is particularly sensitive to composition because it takes into account both $\kappa$ and $c$. Changes in either or both of $\kappa$ and $c$ will typically result in a change in $\sqrt{\rho c \kappa}$. Changes in relative concentrations of different components in a multi-component material can be detected where the different components have different thermal properties. Changes in structure can be detected where there is a density or compositional change.

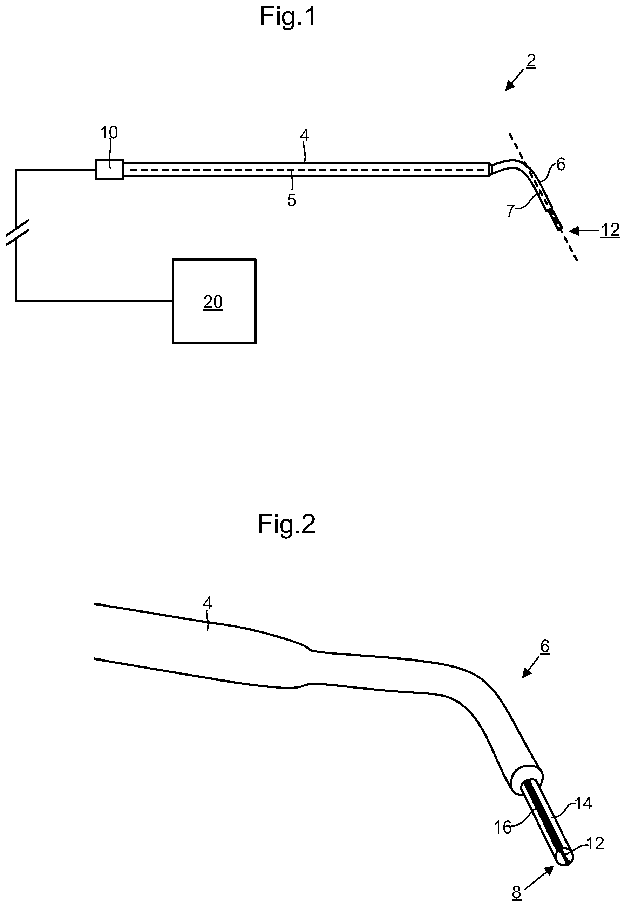

Figures 1 and 2 depict an example dental probe 2 for performing the method. The probe 2 comprises a sensor element 12. The probe 2 comprises a handle 4. The sensor element 12 is mounted distally relative to the handle 4, typically at or near a distal end of the probe 2. The probe 2 is configured (e.g. by adopting a form similar to a conventional explorer probe) to allow a user holding the handle 4 to bring the sensor element 12 into thermal contact with a dental region to be sensed. The sensor element 12 is configured to allow a heating pulse to be applied to the dental region via the sensor element 12. The sensor element 12 is further configured to allow measurement of a response of the sensor element 12 during the heating pulse. The response depends on heat transfer characteristics of the dental region. The response is used to determine chemical or structural information about the dental region.

In the embodiment shown, the sensor element 12 is mounted on a locally elongate member having a longitudinal axis 7 (see Figure 1). The longitudinal axis 7 is not parallel to (e.g. is angled obliquely to) a longitudinal axis 5 of the handle 4. The overall form of the probe 2 can thus be made similar to a conventional explorer probe, allowing efficient access to most regions within the mouth.

In an embodiment, the probe 2 is provided as part of a probe system. The probe system comprises the probe 2 and a measurement unit 20. An example of such a probe system is depicted schematically in Figure 1. Connector element 10 provides a connection between the probe 2 and the measurement unit 20. The connection may be implemented by wires running between the probe 2 and the measurement unit 20, a wireless connection may be used, or the measurement unit 20 and probe 2 may be provided as a single unit (e.g. with the measurement unit 20 provided within the handle 4 of the probe 2).

The measurement unit 20 is configured to apply the heating pulse via the sensor element 12 and to measure the response of the sensor element 12 to the heating pulse. The measurement unit 20 may therefore comprise a power supply and data processing hardware to control the supply of the heating power and to control the measurement process. The measurement unit 20 may be connected to mains power or be powered by a battery (e.g. when the probe and measurement unit 20 are provided as a single wireless unit). The measurement unit 20 may comprise a memory for storing measurements and/or calibration data for analysing measurements.

In an embodiment, the sensor element 12 comprises a resistive element. During use the resistive element is brought into thermal contact with the dental region of interest. The heating pulse is applied by driving an electrical current through the resistive element to create Joule heating. The response of the sensor element 12 during the heating pulse is determined by the measurement unit 20 by measuring an electrical response of the resistive element to the heating pulse. The measured electrical response may be proportional to a resistance of the resistive element or to a quantity that is dependent on the resistance of the resistive element.

In an embodiment, the measurement unit 20 applies a plurality of the heating pulses. Each heating pulse is applied by driving an electrical current through the resistive element. In an embodiment, top hat shaped pulses are applied, but other pulse shapes could be used if desired. In an embodiment, the plurality of heating pulses each have the same duration. The heating pulses are regularly spaced apart from each other (i.e. the spacing between each pair of heating pulses is the same). The duration of each heating pulse is equal to or less than the separation between the heating pulses. This provides time for the resistive element to cool between each heating pulse. In an embodiment, the separation between heating pulses is the same as the duration of each heating pulse. This provides a minimum time for the resistive element to cool between heating pulses, thereby allowing a high measurement sampling rate and, as a consequence, high accuracy (by averaging) and/or time resolution.

The measurement unit 20 measures an electrical response of the resistive element to the heating pulses, for example by measuring a voltage dependent on the resistance of the resistive element and the current being driven through the resistive element. The resistance of the resistive element varies as a function of the temperature of the resistive element. Measuring the electrical response of the resistive element thus corresponds to measuring a temperature response of the resistive element.

The electrical response of the resistive element to the heating pulses can be used to determine chemical and/or structural information about materials adjacent to the resistive element because the variation in the temperature of the resistive element with time will depend on the heat transfer characteristics of those materials.

In an embodiment, a response to the heating pulse is compared with the response to a corresponding heating pulse applied to a reference material. The size of the response, the variation of the response as a function of time, or various other aspects of the response may be considered. Any deviation from the response measured for the reference material may be used to detect a deviation from normality for the dental region being sensed. The nature of the heating pulses may be selected to achieve optimum sensitivity for the particular dental region being measured. This may involve selecting particular pulse shapes, amplitudes, durations and/or repetition rates, for example.

In an embodiment, an example of which is depicted in Figure 2, the resistive element is mounted on a substrate 14 in such a way that at least 10% of the surface area of the resistive element is in contact with the substrate 14, optionally via a support material encapsulating the resistive element (e.g. a thin film of electrically insulating material), optionally more than 30%, optionally around 50%. In an embodiment the resistive element is a thin film resistive element (e.g. thin film resistance thermometer). In an embodiment the resistive element comprises a thin film of platinum or gold mounted on the substrate 14. In an embodiment, the resistive element has a first surface configured to face towards the dental region to be sensed (facing out of the page in Figure 2) and a second surface facing towards the substrate 14 (facing into the page in Figure 2). It is understood that the first and second surfaces are the large surfaces of the thin film (and do not include any of the very thin side surfaces). In an embodiment no portion of the entity being sensed is present between the second surface and the substrate 14. In the particular example of Figures 1 and 2, the resistive element is flat against a distal end of a substantially cylindrical substrate 14. Substantially 50% of the surface of the resistive element is in contact with the substrate 14. In the example shown, electrically conductive tracks 16 are formed on an exterior surface of the substrate 14 to provide the required electrical connections to the resistive element. The presence of the substrate 14 allows relatively large currents to be applied to the resistive element without the resistive element overheating, which could damage the resistive element and/or material that is in contact with the resistive element.

In various embodiments the resistive element is metallic. In these embodiments, the resistive element may be configured such that the thermal contact between the resistive element and the dental region being sensed will not result in a significant reduction in the electrical resistance between one end of the resistive element and the other end of the resistive element. This may be achieved by arranging for the resistivity of the resistive element to be much lower than the resistivity of the entity to be sensed or by positioning a thin layer of electrically insulating material between the resistive element and the entity to be sensed.

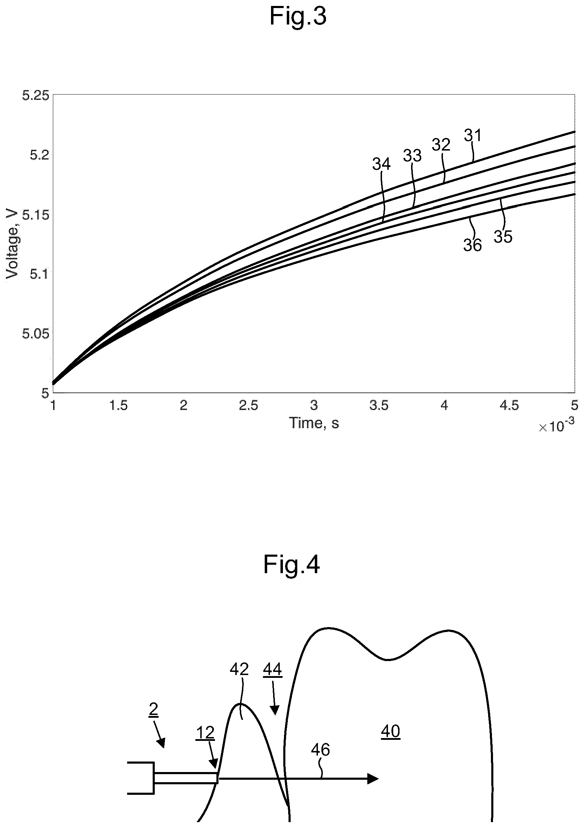

Figure 3 depicts example responses from a sensor element 12 comprising a thin film resistive element. The responses consist of a variation of a voltage across the resistive element during a time interval in which a heating pulse of duration $5 \times 10^{-3}$s is being applied to a dental region. Each of the curves corresponds to a different material potentially present in a dental region, as follows: enamel (31), root (32), air (33), white filling (34), white gold (part of implant) (35), decay (36). The response is distinctly different for each of the different materials. A particularly large differences is seen between the curve for enamel (31) and the curve for decay (36). Decay can thus be detected particularly sensitively.

The method may be applied to dental regions of various types, including any surface or structure relevant to oral health, including one or more regions of one or more of the following: teeth, gums, oral mucosa, upper jaw, lower jaw, tongue, salivary glands, uvula, and frenulum. In one class of embodiments, the dental region comprises a portion of a tooth. The sensor element 12 in such embodiments would be positioned in thermal contact with the tooth during application of the heating pulse. Heat from the heating pulse thus propagates into the portion of the tooth being sensed during the application of the heating pulse. The efficiency with which heat is transferred away from the sensor element 12 depends on heat transfer characteristics of the portion of the tooth. The response of the sensor element 12 (e.g. voltage) thus also depends on the heat transfer characteristics and therefore the chemical and/or structural composition of the portion of the tooth.

When applied to teeth, the sensor element 12 may be configured to perform one or more of the following: detecting transitions with depth from enamel to dentine and from dentine to infected dentine; detecting decay under pits before drilling; detecting depth of fillings; detecting the amount of tooth tissue before a nerve is hit; detecting active and non-active decay; detecting cracks; detecting depth of metal thickness in crowns/inlays; detecting dental decay beneath crowns.

In an alternative embodiment, as depicted in Figure 4, the dental region being sensed comprises a periodontal pocket 44 between a gum 42 and a tooth 40. The sensor element 12 is positioned in thermal contact with the periodontal pocket 44 (e.g. on the outside of a gum adjacent to the periodontal pocket, as shown in Figure 4) during application of the heating pulse such that heat from the heating pulse propagates through the periodontal pocket 44 (indicated by arrow 46) during application of the heating pulse. The response of the sensor element 12 provides information about the size or existence of a gap between the gum 42 and the tooth 40 at the position (e.g. height) of the sensor element 12. The respective heat transfer characteristics of the gum 42, the liquid or other material in the periodontal pocket 44, and the tooth 40 are significantly different to each other. Furthermore, their respective contributions to the response (e.g. voltage) of the sensor element 12 can be distinguished from each other because they will contribute to the response at different times. The gum, being nearest to the sensor element 12, will contribute to the response of the sensor element 12 immediately with substantially no influence from the periodontal pocket 44 or from the tooth 42. When the heating pulse has penetrated through the gum and enters the periodontal pocket 44 a combination of both the gum and the periodontal pocket will contribute to the response of the sensor element 12. When the heating pulse has penetrated through the gum and the periodontal pocket and has entered the tooth 44, a combination of the gum 42, the periodontal pocket 44 and the tooth 44 will contribute to the response of the sensor element 12. The contribution from the periodontal pocket 44 will depend on the position of the sensor element 12. If the sensor element 12 is positioned below the lowest point of the periodontal pocket 44, the periodontal pocket will not contribute significantly to the response of the sensor element 12 to the heating pulse. When the sensor element 12 is at higher levels, the contribution from the periodontal pocket will progressively increase as the gap between the gum 42 and the tooth 40 progressively increases. The probe can thus be used to objectively map the periodontal pocket 44. The sensor element 12 may also be used more generally for assessing gum disease and its severity. Alternatively or additionally, the sensor element 12 may be used to detect the amount of bone present around a tooth and implant. Failure of a dental implant due to bone reduction may be detected at an early stage.

The application to measuring the periodontal pocket 44 is an example of a class of embodiments in which the heat from the heating pulse propagates through plural layers of different structural or chemical composition and the analysis of the response makes it possible to distinguish between contributions from different layers. In embodiments of this type, the response from the sensor element 12 may be analysed to identify one or more target time periods. Each target time period is a time period in which the response to the heating pulse is determined predominantly by a different combination of one or more of the plural layers. In the periodontal pocket example discussed above, three different target time periods can be identified: 1) a first target time period in which only the gum contributes; 2) a second target time period in which only the gum and the periodontal pocket contribute; and 3) a third target time period in which the gum, periodontal pocket and tooth contribute. The same principle applies in other situations where plural layers are provided. For example, when the sensor element 12 is positioned directly against a tooth, plural layers comprising for example an enamel layer, a decay layer, and a nerve layer, may be sensed. The sensor element 12 may thus be used to determine a variation of the thermal properties of the dental region being sensed as a function of distance away from the sensor element 12.

In an embodiment, a coupling fluid or gel is provided between the sensor element and the dental region during application of the heating pulse to the dental region. The coupling fluid or gel helps reproducibly to provide a high quality thermal contact between the sensor element and the dental region being sensed. The coupling fluid or gel will in general have heat transfer characteristics different from those of the dental region being sensed. These different properties make it possible to recognize which part of the response of the sensor element is due solely to the coupling fluid or gel and which part provides information about the dental region being sensed.

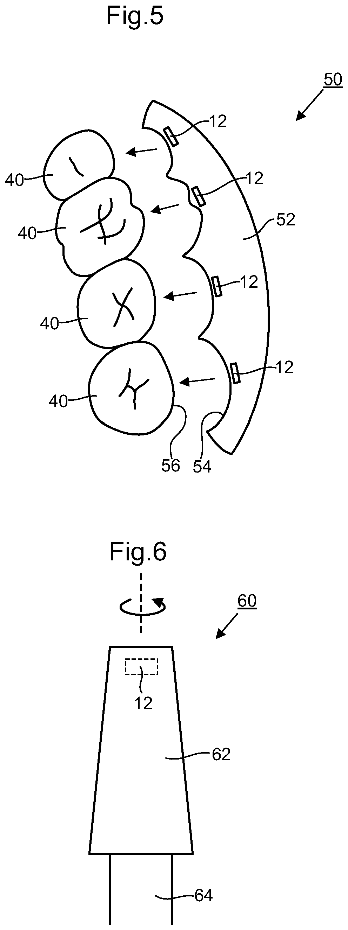

Figure 5 depicts an example of a device 50 for sensing multiple dental regions by simultaneously holding plural sensor elements 12 against a corresponding plurality of different portions of a plurality of teeth 40 (e.g. against different teeth). The device 50 comprises a support structure 52 that fits against the plurality of teeth 40. The support structure 52 comprises a plurality of sensor elements 12. In the embodiment shown, each of the sensor elements 12 is positioned adjacent to a different tooth 40 (when the support structure 52 is fitted against the teeth). Each sensor element 12 allows a heating pulse to be applied to the dental region (e.g. tooth) against which the sensor element 12 is positioned. Each sensor element 12 further allows measurement of a response of the sensor element 12 during the heating pulse to determine chemical or structure information about the dental region (based on the heat transfer characteristics of the dental region). In an embodiment, the support structure 52 comprises a conforming surface 54 that is pre-shaped to conform with an outer profile 56 of the plurality of teeth 40. The support structure 52 is thus personalized to the patient. The support structure 52 may be formed by taking a mould of the patient's teeth using techniques known in the art. Alternatively or additionally, the support structure 52 may comprise a deformable interior surface that conforms with the outer profile 56 of the plurality of teeth 40 when positioned in use against the teeth 40. A force associated with pressing the support structure 52 against the teeth causes the support structure to deform and thereby conform with the outer profile 56. Each of the sensor elements 12 may adopt any of the configurations described in detail above. Each of the sensor elements 12 may be connected to a measurement unit 20 as described above.

In the example shown in Figure 5 the support structure 52 fits against an outer side surface of four teeth. In other embodiments, the support structure 52 fits against less than four or more than four teeth. In other embodiments, the support structure 52 alternatively or additionally supports sensor elements 12 that fit against biting surfaces of teeth and/or against inner side surfaces of teeth. The support structure 52 may for example be configured to fit over the teeth in the manner of a mouth guard so as to be simultaneously in contact with inner side surfaces, outer side surfaces and biting surfaces of teeth.

Providing a support structure that holds plural sensor elements against different portions of teeth allows measurements on teeth to be made reproducibly and conveniently. The support structure can be personalized to each patient. Meaningful comparisons of measurements made at different times can be made, allowing sensitive detection of dental problems at an early stage. Multiple different positions on teeth and/or multiple teeth can be measured simultaneously. In principle a sensor element 12 could be brought into thermal contact individually with each and every tooth of a patient, optionally measuring each tooth at plural different positions. A thorough mapping of a patient's dental state can be carried out quickly and easily. The apparatus is easy enough to use that a patient can perform measurements at home without professional assistance. The patient simply positions the apparatus 50 in the mouth (e.g. by biting on it) and makes an electrical connection with a measurement unit 20 to allow driving of the sensor elements 12. Dental state can be monitored frequently without visiting a dentist, allowing dental conditions to be detected earlier.

Figure 6 depicts an example of a drill 60 using a sensor element 12. The drill 60 comprises a rotatable cutting surface 62. In an embodiment the drill 60 is a dental drill and the rotatable cutting surface 62 is suitable for drilling into teeth. The sensor element 12 is positioned behind the rotatable cutting surface 62 (i.e. so that during cutting the material forming the rotatable cutting surface 62 is between the sensor element 12 and the material being cut). The sensor element 12 is configured to allow a heating pulse to be applied to a region in front of the cutting surface 62 (e.g. above the cutting surface in the orientation shown in Figure 6) during rotation of the cutting surface 62 in use and to allow measurement of a response of the sensor element 12 to the heating pulse during the heating pulse to determine chemical or structural information about the region in front of the cutting surface 62 (the response being dependent on heat transfer characteristics of the region in front of the cutting surface). The nature of material in front of the drill can thus be assessed before it is drilled through. In the context of drilling through teeth, for example, the drill 60 can detect a relative distance between the drill and a nerve within a tooth. The risk of drilling too close to a nerve (which can cause sensitive fillings) can be reduced. The drill 60 may alternatively or additionally detect a depth of decay, so that drilling can be stopped reliably and accurately at an optimal depth. The sensor element 12 may adopt any of the configurations described in detail above. The sensor element 12 may be connected to a measurement unit 20 as described above.

In an embodiment, the cutting surface 62 comprises a layer of diamond, diamond-like carbon, or tungsten carbide and the sensor element 12 is positioned in contact with the layer of diamond, diamond-like carbon or tungsten carbide. The layer of diamond, diamond-like carbon, or tungsten carbide protects the sensor element 12 from damage while also being sufficiently thermally conductive that the heating pulse can easily pass through and sense material beyond the drill tip.

In an embodiment a real time display is provided to show a position of the drill relative to structures of interest with the tooth, such a region of decay, or a nerve. In an embodiment the sensor element 12 is additionally configured to measure a temperature of the region in front of the cutting surface. Excessive heating of the tooth by the drilling process can thus be avoided. Measurement of temperature can be achieved using the sensor element 12, particularly where the sensor element 12 comprises a thin film resistive element such as a platinum thin film thermometer.

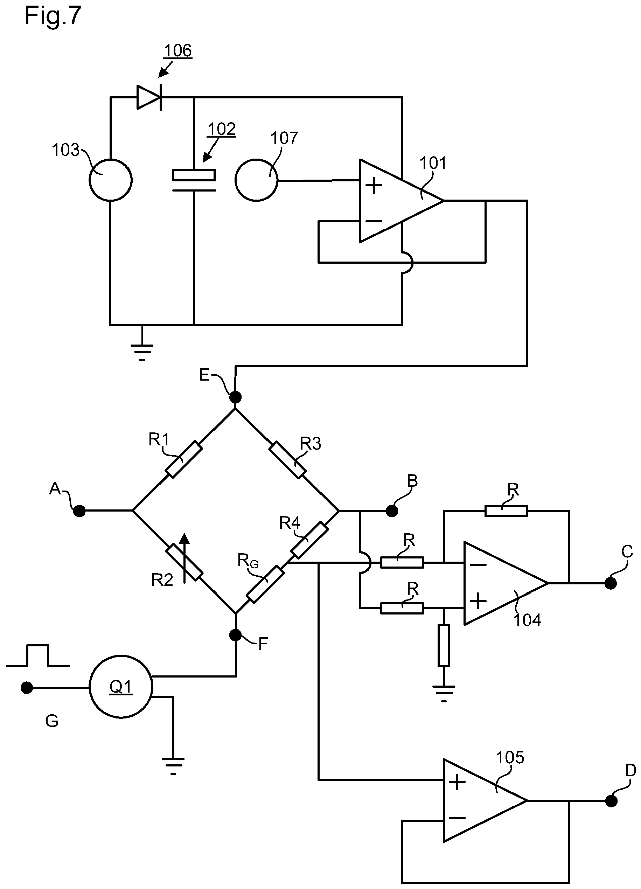

Figure 7 depicts example circuitry for use in the measurement unit 20 for measuring the response of the sensor element 12 to the heating pulses in the case where the sensor element 12 comprises a resistive element. The following elements are shown in Figure 7:

| | |
|---|---|
| 101 | Power amplifier (e.g. about 10A RATED) |
| 102 | Charge store (e.g. about 40,000 $\mu$F) |
| 103 | Power supply (e.g. about 30V DC) |
| 104 | Differential amplifier for $I$ |
| 105 | Buffer amplifier for $V$ |
| R1 + R2 | Bridge balance |
| R3 + $R_G$ | Active bridge half |
| Q1 | Power switch (e.g. fast, low resistance MOSFET) |
| C | Output of current $I$ |
| D | Output of voltage $V$ |
| E | High side of bridge |
| F | Low side of bridge |
| G | Signal pulse control |
| R4 | Current sense shunt (resistance) (e.g. 20 m$\Omega$) |
| A + B | Diagnostic differential signal outputs for development |
| 106 | Diode rectifier |
| 107 | Voltage reference |

A voltage generated by voltage supply 103 is fed through a rectifier diode 106 to charge a high capacity storage 102. The storage 102 provides a high current power source to the power amplifier 101. A voltage reference 107 sets a high side voltage presented at E.

A bridge is created between the points A, E, B and F. In an example, R3 and $R_G$ are about 1.0 Ohms, and R1 and R2 are about 470 Ohms. A power switch device Q1 is provided to rapidly bring point F to ground under a signal pulse at G. The circuit enables a steady bridge voltage to be maintained without demanding a high gain bandwidth from the power amplifier 101. The power amplifier 101 needs only to maintain a DC level. High energy pulses of precise timing are made possible using a fast MOSFET power switch for Q1 at the low side of the bridge.

When the bridge is energised the differential voltage points (A & B) will provide a voltage corresponding to the Ohmic resistance change of the gauge element $R_G$ (e.g. the resistive element of the sensor element 12). The other resistors in the bridge are chosen to have a very low parts-per-million (ppm) change in resistance with temperature. Therefore observed bridge voltages are only a function of the gauge $R_G$.

For precise measurements of heat transfer to the resistive element, and from the resistive element to material in contact with the resistive element, it is desirable to measure the voltage $V$ and current $I$ across the resistive element. The current is determined from the output of the circuit at C. The voltage is determined from the output of the circuit at D. Thus the energy input and the corresponding rise in temperature can be determined and the heat transfer function to the material in contact with the resistive element can be computed.

The total energy and energy rate can be controlled by varying the reference voltage 107 and the pulse duration at G.

The circuit allows a modest power source to store energy to deliver very high energy density pulses. Electronic controls may be provided to activate the power level and pulses duration whilst reading the voltage signals at C and D. The electronic controls may be provided by the measurement system 8 or processing unit 18, or both.

In an embodiment, fast ADC to storage in computer memory is employed leaving time to compute the heat transfer data from which quantitative measurements can be performed and compared to calibrated lookup tables to provide qualitative assessments of the composition of the dental region being sensed.

Embodiment of the disclosure are defined in the following numbered clauses.

1. A method of sensing a dental region, comprising:

using a sensor element to apply a heating pulse to the dental region; and
determining chemical or structural information about the dental region by measuring a response of the sensor element during the heating pulse, the response being dependent on heat transfer characteristics of the dental region.

2. The method of clause 1, wherein the dental region comprises a portion of a tooth.

3. The method of clause 1, wherein the dental region comprises a periodontal pocket, the sensor element being positioned such that heat from the heating pulse propagates through the periodontal pocket during application of the heating pulse.

4. The method of any preceding numbered clause, wherein the sensor element comprises a resistive element and the heating pulse is applied by driving an electrical current through the resistive element.

5. The method of any preceding numbered clause, wherein the sensor element comprises a resistive element and the response of the sensor element comprises an electrical response of the resistive element.

6. The method of clause 4 or 5, wherein the resistive element is mounted on a substrate in such a way that at least 10% of the surface area of the resistive element is in contact with the substrate, optionally via a support material encapsulating the resistive element.

7. The method of clause 6, wherein the resistive element is a thin film resistive element having a first surface configured to face towards the dental region to be sensed and a second surface facing towards the substrate.

8. The method of any preceding numbered clause, wherein heat from the heating pulse propagates through plural layers of different chemical or structural composition and the measured response of the sensor element is analysed to identify one or more target time periods, each target time period being defined as a time period in which the response of the sensor element is determined predominantly by a different combination of one or more of the plural layers.

9. The method of any preceding numbered clause, wherein the determined chemical or structural information comprises a variation as a function of distance from the sensor element of the chemical or structural composition of the dental region.

10. The method of any preceding numbered clause, further comprising providing a coupling fluid or gel between the sensor element and the dental region during application of the heating pulse to the dental region.

11. A dental probe, comprising:
a handle and a sensor element mounted distally relative to the handle, wherein:

the probe is configured to allow a user holding the handle to bring the sensor element into thermal contact with a dental region to be sensed; and
the sensor element is configured to allow a heating pulse to be applied to the dental region via the sensor element and to allow measurement of a response of the sensor element during the heating pulse to determine chemical or structural information about the dental region, the response being dependent on heat transfer characteristics of the dental region.

12. The probe of clause 11, wherein the sensor element is mounted on a locally elongate member having a longitudinal axis that is not parallel to a longitudinal axis of the handle.

13. The probe of clause 11 or 12, wherein the sensor element comprises a resistive element.

14. A dental probe system comprising:

the probe of any of numbered clauses 11-13; and
a measurement unit configured to apply the heating pulse via the sensor element and to measure a response of the sensor element to the heating pulse.

15. The system of clause 14, wherein the measurement unit is configured to:
determine chemical or structural information about the dental region by comparing a measured response of the sensor element during a heating pulse to a stored reference response.

16. A device for sensing multiple dental regions, comprising:
a support structure configured to fit against a plurality of teeth of a patient, wherein:

the support structure comprises a plurality of sensor elements;
each sensor element is configured to be positioned against a respective different dental region when the support structure is positioned in use against the plurality of teeth; and

each sensor element is configured to allow a heating pulse to be applied to the dental region against which the sensor element is positioned and to allow measurement of a response of the sensor element during the heating pulse to determine chemical or structure information about the dental region, the response being dependent on heat transfer characteristics of the dental region.

17. The apparatus of clause 16, wherein the support structure comprises a conforming surface that is pre-shaped to conform with an outer profile of the plurality of teeth.

18. The apparatus of clause 16 or 17, wherein the housing comprises a deformable interior surface configured to conform with an outer profile of the plurality of teeth when positioned in use over the plurality of teeth.

19. The apparatus of any of clauses 16-18, wherein the fit between the support structure and the plurality of teeth is such as to hold the support structure stably in place by friction between the support structure and the plurality of teeth.

20. A drill comprising:

a rotatable cutting surface and a sensor element positioned behind the rotatable cutting surface, wherein:

the sensor element is configured to allow a heating pulse to be applied to a region in front of the cutting surface during rotation of the cutting surface in use and to allow measurement of a response of the sensor element during the heating pulse to determine chemical or structural information about the region in front of the cutting surface, the response being dependent on heat transfer characteristics of the region in front of the cutting surface.

## Claims

1. A drill (60) comprising:

a rotatable cutting surface (62) and a sensor element (12) positioned behind the rotatable cutting surface (62), wherein:

the sensor element (12) is configured to allow a heating pulse to be applied to a region in front of the cutting surface (62) during rotation of the cutting surface (62) in use and to allow measurement of a response of the sensor element (12) during the heating pulse to determine chemical or structural information about the region in front of the cutting surface (62), the response being dependent on heat transfer characteristics of the region in front of the cutting surface (62).

# Fig.1

2

10   4   5   6   7   12   20

# Fig.2

4   6   14   16   12   8

# Fig.3

# Fig.4

# Fig.5

# Fig.6

# Fig.7

Europäisches
Patentamt

European
Patent Office

Office européen
des brevets

# EUROPEAN SEARCH REPORT

Application Number

EP 22 16 7118

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GEORG D. STRBAC ET AL: "Drilling- and Withdrawing-Related Thermal Changes during Implant Site Osteotomies", CLINICAL IMPLANT DENTISTRY AND RELATED RESEARCH, vol. 17, no. 1, 28 February 2015 (2015-02-28), pages 32-43, XP055214930, ISSN: 1523-0899, DOI: 10.1111/cid.12091 * Abstract on first page * ----- | 1 | INV. A61C19/04 A61C3/02 A61B5/053 A61B5/00 |
| A | WO 2016/097723 A1 (ISIS INNOVATION [GB]) 23 June 2016 (2016-06-23) * page 2, lines 8-13 * * last 4 lines * * page 8, paragraph 3 * * page 13, paragraph 3 * * page 14, paragraph 2 * * last 4 lines; page 25 * ----- | 1 | |

TECHNICAL FIELDS
SEARCHED      (IPC)

A61C
A61B

The present search report has been drawn up for all claims

| Place of search | Date of completion of the search | Examiner |
|---|---|---|
| Munich | 30 June 2022 | Kerner, Bodo |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
.......................................................................
& : member of the same patent family, corresponding
    document

EPO FORM 1503 03.82 (P04C01)

## EP 4 042 970 A1

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2022

| Patent document cited in search report | | Publication date | Patent family member(s) | | Publication date |
|---|---|---|---|---|---|
| WO 2016097723 | A1 | 23-06-2016 | CN | 107407649 A | 28-11-2017 |
| | | | EP | 3234559 A1 | 25-10-2017 |
| | | | JP | 6713997 B2 | 24-06-2020 |
| | | | JP | 2017538128 A | 21-12-2017 |
| | | | US | 2017350841 A1 | 07-12-2017 |
| | | | US | 2020225181 A1 | 16-07-2020 |
| | | | WO | 2016097723 A1 | 23-06-2016 |

EPO FORM P0459

For more details about this annex : see Official Journal of the European Patent Office, No. 12/82